# EUROPÄISCHE PATENTSCHRIFT

(11) **EP 2 028 488 B1**
(45) Veröffentlichungstag und Bekanntmachung des Hinweises auf die Patenterteilung: **25.02.2015**
(21) Anmeldenummer: 07113687.3
(22) Anmeldetag: 02.08.2007
(51) Int. Cl.: G01N 33/487

(54) **Transfereinheit für Testelemente**
Transfer unit for test elements
Unité de transfert pour éléments test

(43) Veröffentlichungstag der Anmeldung: 25.02.2009
(73) Patentinhaber: F. Hoffmann-La Roche AG, 4070 Basel (CH); Roche Diagnostics GmbH, 68305 Mannheim (DE)
(72) Erfinder: List, Hans, 64754 Hesseneck-Kailbach (DE)
(74) Vertreter: Stößel, Matthias

(56) Entgegenhaltungen:
- EP-A- 1 367 339
- WO-A2-2006/058653
- US-A1- 2002 057 993
- US-A1- 2007 173 739

## Beschreibung

### Technisches Gebiet

Die Erfindung bezieht sich auf eine Transfereinheit für Testelemente, die insbesondere zum Nachweis eines Analyten in einer Körperflüssigkeit eingesetzt werden.

### Stand der Technik

Systeme und Geräte zur Analyse von Körperflüssigkeiten sind vielfältig bekannt, insbesondere solche Systeme, in denen eine Vielzahl von Testelementen bevorratet ist. Die Testelemente werden im Allgemeinen streifenförmig ausgebildet und sind in einem Magazin untergebracht. Zur Magazinierung von Testelementen sind scheibenförmige Aufbewahrungssysteme oder trommelförmige Aufbewahrungssysteme geeignet. Scheibenbeziehungsweise trommelförmig ausgebildete Magazinierungen sind jedoch nur für eine geringe Anzahl von 5 bis 20 Testelementen praktikabel. Bis zu dieser Anzahl von Testelementen bieten ein scheibenförmiges Magazin und ein trommelförmiges Magazin eine gute Möglichkeit zur Platz sparenden Aufbewahrung von Testelementen.

Bei größeren Stückzahlen von aufzubewahrenden Testelementen wird ein Scheibenmagazin beziehungsweise ein Trommelmagazin jedoch unhandlich groß, da zur Aufnahme von einer größeren Anzahl von Testelementen der Durchmesser des Scheibenmagazins beziehungsweise der Durchmesser des Trommelmagazins zu erhöhen ist. Für Testelementvorräte von 20 Stück und mehr können gemäß US 6,827,899 Stapelmagazine eingesetzt werden.

Das aus US 6,827,899 bekannte Stapelmagazin hält frische Testelemente bereit, überlässt dem Anwender jedoch die Entsorgung einzelner gebrauchter Testelemente, die mit einer Körperflüssigkeit kontaminiert sind.

US 6,159,424 bezieht sich auf eine Einrichtung zur Handhabung von Messstreifen, mit denen Flüssigkeitsproben genommen werden, so zum Beispiel Blutproben. Die Messstreifen weisen ein poröses Material zum Absorbieren der Flüssigkeitsprobe sowie zu deren Analyse auf. Die Messeinrichtung gemäß US 6,159,424 umfasst einen Speicher für Messstreifen, die Proben aufnehmen, der als Verbundkonstruktion ausgebildet ist und einen weiteren Speicher zur Aufnahme gebrauchter Messstreifen sowie einen Messstreifen-Zuführmechanismus umfasst. Die aus US 6,159,424 bekannte Einrichtung mit dem Speicher für ungebrauchte Messstreifen und dem Speicher für gebrauchte Messstreifen wird bevorzugt innerhalb eines Gehäuseteils integriert.

US 6,534,017 B1 bezieht sich auf eine Speichereinrichtung für Testelemente. Die Testelemente werden innerhalb eines Magazins aufgenommen, wobei die Testelemente eine oder mehrere Testzonen aufweisen, die nebeneinanderliegend auf einem rechteckförmig ausgebildeten Träger angeordnet sind. Das Magazin umfasst zumindest ein Paar von Führungsnuten, welche einander gegenüberliegend angeordnet sind und in welche die Testelemente eingeführt werden, derart, dass diese direkt nebeneinander liegen und die Kanten aneinanderliegender Träger einander anstoßen. Gemäß eines weiteren Aspekts der US 6,534,017 B1 wird ein Schlitten vorgeschlagen, welcher zusätzlich zum Magazin gemäß US 6,534,017 B1 eingesetzt wird und dazu dient, eine Schicht von Testelementen entlang der Führungsnuten zum gegenüberliegenden Ende zu bewegen und Testelemente aus dem Magazin auszugeben.

Nachteilig bei den vorstehend skizzierten Lösungen gemäß des Standes der Technik ist der Umstand, dass der Anwender eines Analysesystems beziehungsweise eines Analysegerätes zur Untersuchung einer menschlichen Körperflüssigkeit auf einem Analyten, die Entsorgung kontaminierter Testelement selbst vorzunehmen hat. Dieser Umstand wird als höchst unbefriedigend empfunden, so dass Abhilfe zu schaffen ist.

### Darstellung der Erfindung

Der vorliegenden Erfindung liegt die Aufgabe zugrunde, eine Transfereinrichtung bereitzustellen, welcher ein Testelement, welches zum Beispiel streifenförmig ausgebildet sein kann, einem Frischvorrat entnimmt, einer Bearbeitungsposition zuführt und in einen Abfallbehälter für kontaminierte Testelemente fördert.

Erfindungsgemäß wird diese Aufgabe durch die Merkmale des Patentanspruchs 1 gelöst. Vorteilhafte Weiterbildungen, welche einzeln oder in Kombination verwirklicht sein können, sind in den abhängigen Ansprüchen dargestellt. Sämtliche Ansprüche werden hiermit zum Inhalt der Beschreibung gemacht.

Die erfindungsgemäß vorgeschlagene Lösung zeichnet sich vor allem dadurch aus, dass diese unter Verzicht auf einen elektrischen oder pneumatischen Antrieb oder dergleichen betrieben werden kann. Die Transfereinrichtung ist so einfach aufgebaut, dass sie sich durch einfache Betätigung eines Drehknopfes oder Hebels, mal zur einen Seite, mal zur anderen Seite, betätigen lässt. Für ein vollautomatisches System, das dem Anwender auch diese Arbeit abnimmt, genügt dann jedweder Aktuator, der diese kurzen Schwenkbewegungen ausführen kann.

Zum Antrieb der erfindungsgemäß vorgeschlagenen Transfereinheit zur Förderung von Testelementen in eine Bearbeitungsposition und aus einer Bearbeitungsposition in einen Abfallbehälter reicht die Spannungsquelle aus, die ohnehin bereits an einem Analysegerät zur Analyse von Körperflüssigkeiten vorhanden ist. Zum Auslösen einer Schwenkbewegung eines Transferelementes der erfindungsgemäß vorgeschlagenen Transfereinheit können z.B. Formgedächtnisdrähte eingesetzt werden, die mit der Spannungsquelle des Analysegerätes oder des Analysesystems verbunden werden können. Bevorzugt werden zwei im Wesentlichen parallel zueinander angeordnete Formgedächtnisdrähte eingesetzt, die, falls von einem Strom durchflossen, über ihre Transformationstemperatur erwärmt werden und sich demzufolge auch gegen größere Kräfte zusammenziehen. Bei Abschaltung der Spannung kühlen sie wieder ab und ändern daraufhin ihren E-Modul, wodurch sie mit kleinen Kräften dehnbar werden.

Die erfindungsgemäß vorgeschlagene Transfereinheit umfasst ein im Wesentlichen halbzylinderförmig ausgebildetes Transferelement. Das Transferelement ist um eine Schwenkachse bewegbar. An die Umfangsfläche des Transferelements ist ein Frischvorrat von Testelementen angestellt. Der Frischvorrat von Testelementen kann zum Beispiel eine Anzahl stapelförmig übereinanderliegend angeordneter Testelemente aufweisen. Diese sind, über ein Federelement beaufschlagt, in einem hülsenförmigen Behälter untergebracht. Die Stirnseite des hülsenförmigen Behälters, in dem die unverbrauchten Testelemente aufgenommen sind, ist mit einer Elastomerdichtung versehen, die an die Umfangsfläche des Transferelements der Transfereinheit angestellt ist. Dadurch wird die Kontamination des Frischvorrates von unverbrauchten Testelementen durch Luftfeuchtigkeit vermindert. Aufgrund der Federbeaufschlagung des stapelförmig angeordneten Frischvorrats unverbrauchter Testelemente ist sichergestellt, dass an der Umfangsfläche des Transferelementes der Transfereinheit stets ein frisches, unverbrauchtes Testelement zur Nutzung zur Verfügung steht.

In einer Winkellage zum Frischvorrat ist der Umfangsfläche des Transferelementes der Transfereinheit ein Abfallbehälter zugeordnet. Der Abfallbehälter umfasst an seiner Oberseite einen Abstreifer, mit welchem bei Verschwenken des Transferelementes um die Schwenkachse ein in einer Bearbeitungsposition an der Umfangsfläche des Transferelementes aus dieser zu entfernendes kontaminiertes Testelement von der Umfangsfläche des Transferelementes entfernt und in den Abfallbehälter gefördert wird.

Auch die Re-Magazinierung kontaminierter Testelemente im Abfallbehälter erfolgt stapelförmig, da die von der Umfangsfläche des Transferelementes sukzessive nach Gebrauch abgestreiften kontaminierten Testelemente stapelförmig in den Abfallbehälter gefördert werden, in dem ein weiteres Federelement aufgenommen ist. Entlang eines Umfangsabschnittes umfasst das Transferelement eine Aussparung, innerhalb der sich der mindestens eine Abstreifer bewegt, der die kontaminierten Testelemente aus ihrer Bearbeitungsposition entfernt und dem Abfallbehälter zuleitet.

Der Frischvorrat, in dem die unverbrauchten Testelemente stapelförmig bevorratet werden, ist einerseits durch ein Federelement beaufschlagt und stets an die Umfangsfläche des Transferelements angestellt und wird andererseits durch einen gekröpften Hebel beaufschlagt, der mit einem Anschlag zusammenwirkt. An dem gekröpft ausgebildeten Hebel sind ein erster Formgedächtnisdraht sowie einer zweiter Formgedächtnisdraht befestigt, so dass sie mit annähernd dem gleichen Radius zum Hebeldrehpunkt angreifen. Die jeweils anderen Enden der Formgedächtnisdrähte, die im Wesentlichen parallel zueinander verlaufen, sind links und rechts vom Drehpunkt eines Mitnehmers befestigt, der das Transferelement des Teststreifenmagazins bewegt. Wird einer der Drähte aufgeheizt, so zieht er sich zusammen, verschwenkt dabei den Mitnehmer, wobei der ungeheizte Draht gedehnt wird, und bewegt dabei das Transferelement so, dass die Aufnahmenut für den zu bearbeitenden Teststreifen vor den Frischvorrat bzw. vor den Abfallbehälter gestellt wird.
Sind beide Drähte unbeheizt, so wirkt eine Drehfeder mit Momentensprung so auf den Mitnehmer ein, dass das Transferelement in der Bearbeitungsposition stehen bleibt.

Bei den im Frischvorrat bevorrateten Testelementen handelt es sich bevorzugt um streifenförmig ausgebildete Testelemente. Es ist unerheblich, ob in diesen streifenförmig ausgebildeten Testelementen eine Lanzette oder ein Sampler integriert ist oder nicht. Die erfindungsgemäß vorgeschlagene Transfereinheit zur Förderung von Testelementen umfasst im Wesentlichen den Frischvorrat, den Abfallbehälter sowie die Transfereinheit. Die genannten Bauteile können sowohl sämtlich Bestandteile eines Analysegerätes oder Bestandteile eines Testelemente bevorratenden Magazins sein oder auch auf das Magazin und das Analysegerät verteilt angeordnet sein. Beliebige Kombinationen in der Aufteilung der Komponenten sind demnach möglich.

Vorzugsweise sind alle Bestandteile des Bewegungsapparates gerätefest angeordnet, so dass das disposible Teststreifenmagazin nur den Frischvorrat mit Hülse und Dichtung, das Transferelement und den Abfallbehälter umfasst.

### Zeichnung

Anhand der Zeichnung wird die Erfindung nachstehend eingehender beschrieben.

Es zeigt:
- Figur 1: Eine schematische Darstellung der erfindungsgemäß vorgeschlagenen Transfereinheit mit den Baugruppen Frischvorrat, Abfallbehälter und Transferelement und
- Figur 2: die in Figur 1 dargestellte Transfereinheit mit den Baugruppen Frischvorrat, Abfallbehälter, Transferelement beaufschlagenden Federelementen sowie mit einem gekröpft ausgebildeten Betätigungshebel verbundene Formgedächtnisdrähte.

### Ausführungsvarianten

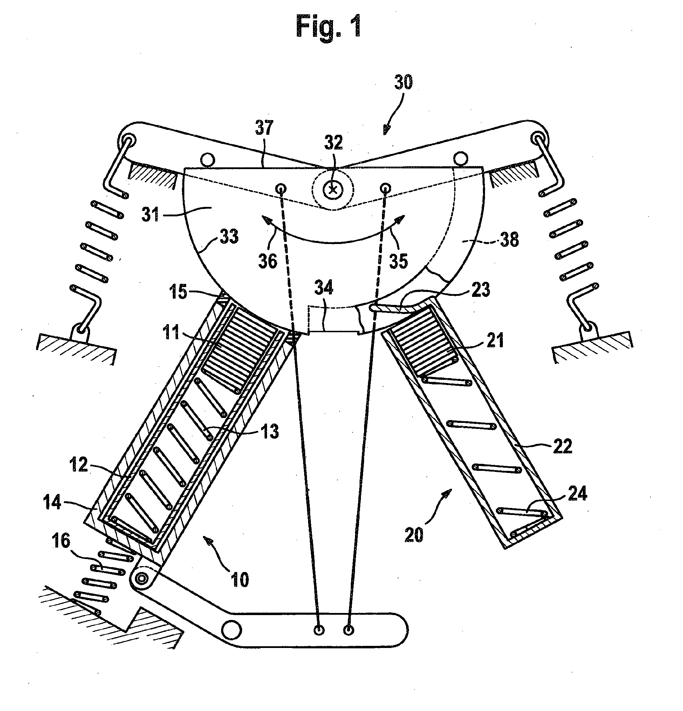

Der Darstellung gemäß Figur 1 sind die wesentlichen Komponenten der erfindungsgemäß vorgeschlagenen Transfereinheit für Testelemente, ein Frischvorrat, ein Transferelement sowie ein Abfallbehälter zu entnehmen.

Figur 1 zeigt, dass ein Frischvorrat 10 eine Hülse 14 umfasst. Innerhalb der Hülse 14 befindet sich ein Rahmen 12 oder ein dosenförmig ausgebildeter Behälter, in dem Testelemente 11 stapelförmig bevorratet sind. Die Testelemente 11, die mit ihren Längsflächen aneinander liegen, werden über ein erstes Federelement 13, welches sich am Boden des Rahmens 12 oder einer Dose abstützt beaufschlagt und an eine Umfangsfläche 33 eines Transferelementes 31 angestellt. Der Rahmen beziehungsweise die Dose ist von einer Hülse 14 übergriffen, an deren offenem, der Umfangsfläche 33 des Transferelementes 31 zuweisender Seite eine Elastomerdichtung 15 angebracht ist. Die Hülse 14 wird über ein zweites Federelement 16, welches sich an einer Abstützfläche abstützt, beaufschlagt, so dass die Elastomerdichtung 15 kontinuierlich gegen die Umfangsfläche 33 des Transferelementes 31 gedrückt wird, wodurch die streifenförmig ausgebildeten Testelemente 11 gegen schädliche Einflüsse von außen, z.B.Feuchtigkeit, geschützt sind.

In einer Winkellage versetzt - in Bezug auf die Umfangsfläche 33 des Transferelementes 31 - befindet sich ein Abfallbehälter 20. In dem Abfallbehälter 20 werden verbrauchte Testelemente 21 aufgenommen. Die Re-Magazinierung der verbrauchten Testelemente 21 innerhalb des Abfallbehälters 20 erfolgt ebenfalls in Stapelform. Auch der Vorrat an verbrauchten Testelementen 21 ist im Abfallbehälter 20 durch ein drittes Federelement 24 beaufschlagt, durch welches die Stapelform im Abfallbehälter 20 aufrechterhalten wird. An der Oberseite des Abfallbehälters 20 befindet sich mindestens ein Abstreifelement 23, welches in eine am Umfang 33 des Transferelementes 31 ausgebildete Aussparung 38 eingreift. Das Transferelement 31 ist auf einer Schwenkachse 32 drehbar gelagert. Bei einer Schwenkbewegung des Transferelementes 31 um die Schwenkachse 32 in eine erste Drehrichtung 35 wird über das mindestens eine Abstreifelement 23 ein zuvor in eine Aussparung 34 eingebrachter bearbeiteter, d.h. verbrauchter Teststreifen 21 aus der Aussparung 34 entfernt und in den Abfallbehälter 20 befördert.

Das Transferelement 31 ist sowohl in die bereits erwähnte Drehrichtung 35 als auch im Uhrzeigersinn 36, d.h. in eine zweite Drehrichtung 36, verschwenkbar. Das Transferelement wird mittels einer Drehfeder mit Momentensprung in einer Mittellage gehalten. Diese Drehfeder, die in der Nullstellung von einem linksdrehenden auf ein rechtsdrehendes Moment springt, kann auf verschiedene Weise aufgebaut sein. Der Übersichtlichkeit halber ist hier die folgende Darstellung gewählt, die beispielhaft, aber nicht limitierend ist.

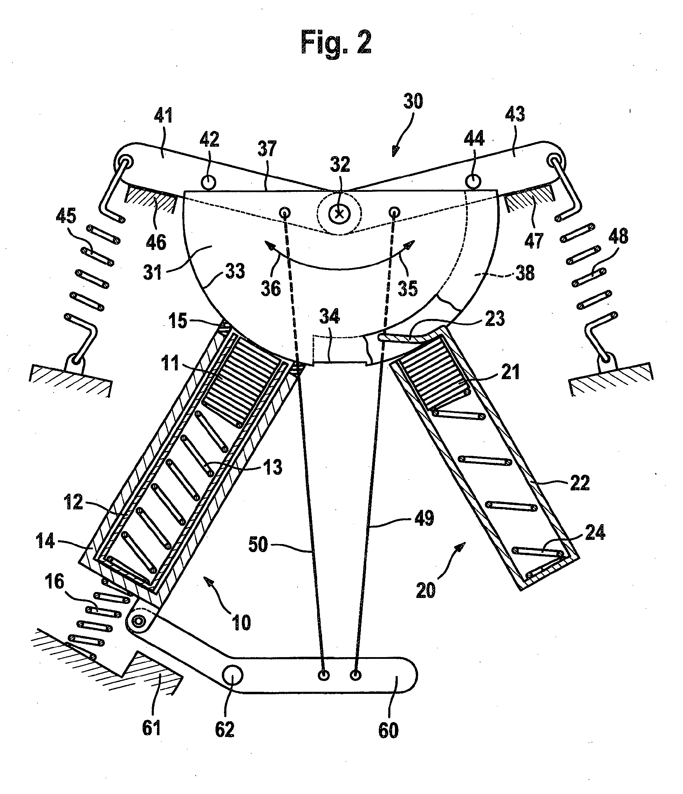

Aus der Darstellung gemäß Figur 2 gehen Federelemente hervor, welche die einzelnen Komponenten der Transfereinheit, einen Frischvorrat, ein Transferelement sowie einen Abfallbehälter umfassend, beaufschlagen beziehungsweise betätigen.

Der Darstellung gemäß Figur 2 ist entnehmbar, dass auf der Schwenkachse 32, um die das halbkreisförmig ausgebildete Transferelement 31 verschwenkbar ist, zwei Hebel 41, 43 aufgenommen sind.

Die beiden Hebel 41 und 43 werden durch Federn 45 und 48 gegen Anschläge 46 und 47 gezogen. Sie tragen Mitnehmer 42 und 44, die an der Kante 37 des Transferelementes 38 anliegen. Wird nun das Transferelement aus seiner Stellung z.B. gegen den Uhrzeigersinn gedreht, so wird der Hebel 43 von seinem Anschlag 47 abgehoben und die Federkraft stützt sich über den Mitnehmer 44 so an der Kante 37 des Transferelementes ab, dass sofort ein Drehmoment definierter Höhe gegen die Drehbewegung wirkt. Bei Drehung im Uhrzeigersinn bewirken die spiegelbildlich angeordneten Hebel, Federn und Anschläge das selbe in der Gegenrichtung. Damit ist sichergestellt, dass das Transferelement stets auch gegen Reibung und Restkräfte in den Aktuatordrähten 49 und 50 in die Bearbeitungsstellung zurückkehrt.

Unterhalb des Frischvorrats 10 befindet sich ein weiterer Hebel 60, der um eine weitere Schwenkachse 62 verschwenkbar ist. Dieser Hebel 60 arbeitet mit einem Anschlag 61 zusammen und ist mit dem unteren Ende der Hülse 14 des Frischvorrates 10 in Eingriff, der zudem konstant durch das zweite Federelement 16 in Anstellrichtung an die Umfangsfläche 33 des Transferelementes 31 beaufschlagt ist.

Am Hebel 60 sind ein erster Formgedächtnisdraht 49 sowie ein zweiter Formgedächtnisdraht 50 befestigt. Es können statt gestreckter Drähte z.B. auch gewickelte Federn aus Formgedächtnislegierung sein. Diese Federn können sinngemäß statt auf Zug auch auf Druck arbeiten. Dafür muss natürlich der Drehpunkt des Hebels 60 so verlegt werden, dass die Öffnungsfunktion des Frischvorrates gegeben ist. Der erste und der zweite Formgedächtnisdraht 49, 50 greifen im wesentlichen mit dem selben Radius am Hebel 60 an Die anderen Enden der Formgedächtnisdrähte 49, 50 sind - beidseits der Schwenkachse 32 - am Transferelement 31 bzw. einem Mitnehmer, der dieses bewegt, befestigt.

Soll das Transferelement 31 verdreht werden, so dass aus dem Frischvorrat 110 ein unverbrauchtes Testelement 11 entnommen wird, so wird der erste Formgedächtnisdraht 49. bestromt, woraufhin er sich zusammenzieht. Diese Kontraktion des ersten Formgedächtnisdrahts 49 führt zunächst zu einer Auslenkung des Hebels 60 entgegen der Wirkung des Federelementes 16. Dadurch wird die Hülse 14 des Frischvorrates 10 von der Umfangsfläche 33 des Transferelementes 31 weggezogen und der Kontakt der Elastomerdichtung 15 zur Umfangsfläche 33 des Transferelementes 31 aufgehoben. Am Ende dieses kurzen Hubweges setzt der Hebel 60 auf dem Anschlag 61 auf, so dass eine weitere Kontraktion des ersten Formgedächtnisdrahtes 49 das Transferelement 31 in Richtung des Uhrzeigersinns 36 auslenkt. Gelangt im Wege dieser Auslenkung die in der Umfangsfläche 33 ausgebildete Aussparung 34 über den Frischvorrat 10, so drückt das erste Federelement 13 ein unverbrauchtes Testelement 11 in die Aussparung 34 hinein.

Wird die Bestromung des ersten Formgedächtnisdrahtes 49 anschließend aufgehoben, so kühlt dieser ab und wird wieder dehnbar, sobald seine Temperatur unter die Transformationstemperatur abgesunken ist. Das Federelement 45, welches über den Hebel 41 und den an diesem befestigten Mitnehmer 42, der auf die Kante 37 des in die zweite Drehrichtung 36 ausgelenkten Transferelementes 31 wirkt, stellt das Transferelement 31 gegen den Uhrzeigersinn 35 wieder in seine in Figur 2 dargestellte Ruhelage. Nun befindet sich das in der Aussparung 34 befindliche Testelement 11 in seiner Bearbeitungsposition. Das in der Aussparung 34 aufgenommene, unverbrauchte Testelement 11 kann nun verwendet werden. Parallel dazu ist der erste Formgedächtnisdraht 49 soweit relaxiert, dass durch das zweite Federelement 16, die Hülse 14 des Frischvorrates 10 wieder gegen die Umfangsfläche 33 des Transferelementes 31 gestellt ist und die an der Öffnungsseite der Hülse 14 angeordnete Elastomerdichtung 15 den Vorrat an unverbrauchten Testelementen 11 abschließt.

Soll das in der Aussparung 34 aufgenommene, nun mit einer Körperflüssigkeit kontaminierte Testelement, welches nunmehr ein verbrauchtes Testelement 21 darstellt, aus der Aussparung 34 entfernt werden, so wird der zweite Formgedächtnisdraht 50 bestromt. Aufgrund der Kontraktion des zweiten Formgedächtnisdrahtes 50 wird der Hebel 60 wieder um die Schwenkachse 62 ausgelenkt und die Elastomerdichtung 15 entgegen der Wirkung des zweiten Federelementes 16 von der Umfangsfläche 33 des halbkreisförmig ausgebildeten Transferelementes 31 abgestellt. Durch die weitere Kontraktion des zweiten Formgedächtnisdrahtes 50 erfolgt eine Verdrehung des Transferelementes 31 gegen den Uhrzeigersinn 35, so dass sich der in der Aussparung 34 am Umfang des Transferelementes 31 aufgenommene, verbrauchte Teststreifen 21 in Richtung des mindestens einen Abstreifelementes 23 bewegt. Das mindestens eine, an der Öffnung des Abfallbehälters 20 aufgenommene Abstreifelement 23 bewegt sich in einer dafür vorgesehenen Aussparung 38 der Umfangsfläche 33 des Transferelementes 31 und fordert den in der Aussparung 34 des Transferelementes 31 aufgenommenen Teststreifen in den Abfallbehälter 20. Innerhalb des Abfallbehälters 20 werden die verbrauchten Testelemente 21 stapelförmig gesammelt und über das innerhalb des Abfallbehälters 20 aufgenommene dritte Federelement 24 in Stapelform gehalten.

Wird die Bestromung des zweiten Formgedächtnisdrahtes 50 unterbrochen, kühlt dieser ab, unterschreitet seine Transformationstemperatur und wird wieder dehnbar. Über das Federelement 48, welches mit dem Hebelende des Hebels 43 verbunden ist sowie den am Hebel 43 aufgenommenen Mitnehmer 44, der auf die Kante 37 des Transferelementes 31 einwirkt, wird dieses wieder in seine in Figur 2 dargestellte Ruhelage bewegt. Bei einer weiteren Relaxation des zweiten Formgedächtnisdrahtes 50 wird die Hülse 14 des Frischvorrates 10 samt darin aufgenommenem Rahmen 12 beziehungsweise Dose 12 über das zweite Federelement 16 wieder an die Umfangsfläche 33 des halbkreisförmig ausgebildeten Transferelementes 31 angestellt, so dass die Elastomerdichtung 15 den Vorrat von in Stapelform aufgenommenen unverbrauchten Testelementen 11 innerhalb des Frischvorrates 10 abschließt.

Diese Anordnung sorgt dafür, dass stets zuerst die Dichtung 15 von der Mantelfläche 33 des Transferelements 31 abgehoben wird, bevor sich das Transferelement 31 aus seiner Ruhelange bewegt. Ebenso ist immer das Transferelement wieder in der Ruhelage angekommen, bevor die Dichtung 15 die Mantelfläche 33 berührt. Auf diese Weise werden sowohl der Kraftbedarf als auch der Verschleiß an der Dichtung minimiert. Zu diesem Zweck sind natürlich die Kenndaten der Aktoren und die Federkräfte aufeinander abzustimmen, was einem Fachmann auf dem Gebiet der Gerätetechnik geläufig ist.

Als Antrieb für die erforderlichen Bewegungen können natürlich auch ein kombiniertes Nockengetriebe, Hubmagnete oder ähnliche geeignete Mittel eingesetzt werden. Da es sich aber bei jeder Bewegung immer nur um einen kurzzeitigen Schaltvorgang handelt, ist ein Paar einfacher Formgedächtnisaktoren zu bevorzugen.

In Abwandlung der in Figur 2 dargestellten Ausführungsvariante des Frischvorrates 10 kann dieser auch ohne die Hülse 14 betrieben werden, was vorliegend jedoch nicht beansprucht wird. In diesem Falle ist der Rahmen 12 beziehungsweise die Aufnahmestruktur für die unverbrauchten Testelemente offen, so dass entweder eine Abdichtung des Gesamtsystems oder gegen Umwelteinflüsse unempfindliche Testelemente einzusetzen sind. In diesem Fall können die Aktoren für den Transfer von Teststreifen auch direkt Drehmoment erzeugende Gebilde sein, wie z,.B. Spiralfedern, Schenkelfedern o.ä.

Abweichend von der in Figur 1 und 2 dargestellten Ausführungsmöglichkeit der Transfereinheit 30, die im Wesentlichen das Transferelement 31, den Frischvorrat 10 sowie den Abfallbehälter 20 umfasst, können die dargestellten Antriebe und ihre zugehörigen Hebelmechanismen 41, 43, 60 feste Bestandteile des Analysegerätes sein. Demgegenüber können der Frischvorrat 10, der Abfallbehälter 20 und das Transferelement 31 Bestandteile eines aus dem Analysegerät entnehmbaren Magazins sein, oder verteilt auf Analysegerät oder Magazin angeordnet sein.

### Bezugszeichenliste

| | | | |
|---|---|---|---|
| 10 | Frischvorrat | 51 | erster Anlenkpunkt von 49 |
| 11 | unverbrauchte Testelemente | 52 | zweiter Anlenkpunkt von 49 |
| 12 | Rahmen | 53 | erster Anlenkpunkt von 50 |
| 13 | erstes Federelement | 54 | zweiter Anlenkpunkt von 50 |
| 14 | Hülse | | |
| 15 | Elastomerdichtung | 60 | dritter Hebel |
| 16 | zweites Federelement | 61 | dritter Anschlag |
| | | 62 | Schwenkachse dritter Hebel |
| 20 | Abfallbehälter | | |
| 21 | verbrauchte Testelemente | | |
| 22 | Behälter | | |
| 23 | Abstreifelement | | |
| 24 | drittes Federelement | | |
| | | | |
| 30 | Transfereinheit | | |
| 31 | Transferelement | | |
| 32 | Schwenkachse | | |
| 33 | Umfangsfläche | | |
| 34 | Aussparung | | |
| 35 | erste Drehrichtung | | |
| 36 | zweite Drehrichtung | | |
| 37 | Kante Transfereinheit 31 | | |
| 38 | Aussparung für Abstreifer | | |
| | | | |
| 41 | erster Hebel | | |
| 42 | Mitnehmer | | |
| 43 | zweiter Hebel | | |
| 44 | Mitnehmer | | |
| 45 | viertes Federelement | | |
| 46 | erster Anschlag | | |
| 47 | zweiter Anschlag | | |
| 48 | fünftes Federelement | | |
| 49 | erster Formgedächtnisdraht | | |
| 50 | zweiter Formgedächtnisdraht | | |

## Patentansprüche

1. Analyseeinrichtung mit einem Analysegerät zur Analyse von Körperflüssigkeiten und einem Magazin für Testelemente (11, 21) mit einem Frischvorrat (10) und einer Transfereinheit (30), die ein Transferelement (31) umfasst, an dessen Umfangsfläche (33) mindestens eine Aussparung (34) zur Aufnahme eines Testelementes (11, 21) ausgebildet ist, wobei ein Abfallbehälter (20) vorgesehen ist, in den verbrauchte Testelemente (21) nach Benutzung re-magaziniert werden, wobei das Transferelement (31) eingerichtet ist, um ein Testelement (21) dem Frischvorrat (10) zu entnehmen, einer Bearbeitungsposition zuzuführen und in den Abfallbehälter (20) zu befördern, **dadurch gekennzeichnet, dass** die Analyseeinrichtung derart eingerichtet ist, dass das Transferelement (31) automatisch nur dann aus der Ruhelage bewegt wird, wenn zuvor eine Dichtung (15) des Frischvorrates (10) abgehoben wurde, wobei sich die Dichtung (15) auf einer Stirnseite eines hülsenförmigen Behälters befindet, in dem der Frischvorrat (10) aufgenommen ist, wobei die Dichtung (15) an die Umfangsfläche (33) des Transferelements (31) angestellt ist.

2. Analyseeinrichtung gemäß Anspruch 1, **dadurch gekennzeichnet, dass** der Frischvorrat (10), der Abfallbehälter (20) und die Transfereinheit (30) Bestandteile des Analysegerätes sind.

3. Analyseeinrichtung gemäß Anspruch 1, **dadurch gekennzeichnet, dass** der Frischvorrat (10), der Abfallbehälter (20) und das Transferelement (31) Bestandteile eines auswechselbaren Magazins sind.

4. Analyseeinrichtung gemäß Anspruch 1, **dadurch gekennzeichnet, dass** der Abfallbehälter (20) an seiner offenen Seite mindestens ein Abstreifelement (23) zum Ausheben eines verbrauchten Testelements (21) aus seiner Bearbeitungsposition (34) aufweist.

5. Analyseeinrichtung gemäß Anspruch 5, **dadurch gekennzeichnet, dass** das Abstreifelement (23) in eine an der Umfangsfläche (33) des Transferelementes (31) ausgebildete Ausnehmung (38) eintaucht.

6. Analyseeinrichtung gemäß einem der vorhergehenden Ansprüche, **dadurch gekennzeichnet, dass** das Transferelement (31) um eine Schwenkachse (32) verschwenkbar ist und durch eine Feder (45, 48) mit Momentensprung in Ruhestellung gehalten wird.

7. Analyseeinrichtung gemäß dem vorhergehenden Anspruch, **dadurch gekennzeichnet, dass** die Ruhestellung des Transferelementes (31) der Transfereinheit (30) durch eine Position definiert ist, in der eine Aussparung (34) zur Aufnahme eines Testelementes (11) in einer Bearbeitungsposition zwischen der Öffnung des Frischvorrates (10) und der Öffnung des Abfallbehälters (20) liegt.

8. Analyseeinrichtung gemäß einem oder mehreren der vorhergehenden Ansprüche, **dadurch gekennzeichnet, dass** die Transfereinheit (30) mittels Aktuatoren (49, 50) angetrieben wird, welche eine Formgedächtnislegierung aufweisen.

9. Analyseeinrichtung gemäß einem der vorhergehenden Ansprüche, **dadurch gekennzeichnet, dass** die Analyseeinrichtung derart eingerichtet ist, dass der Frischvorrat (10) von Aktuatoren (49, 50) geöffnet wird, die auch das Transferelement (31) antreiben.

10. Analyseeinrichtung gemäß einem der vorhergehenden Ansprüche, **dadurch gekennzeichnet, dass** die Analyseeinrichtung derart eingerichtet ist, dass die Dichtung (15) automatisch erst dann wieder auf der Umfangsfläche (33) aufsetzt, wenn sich das Transferelement (31) wieder in einer Ruhestellung befindet.

11. Analyseeinrichtung gemäß einem der vorhergehenden Ansprüche, **dadurch gekennzeichnet, dass** die Analyseeinrichtung derart eingerichtet ist, dass das Transferelement (31) um seine Schwenkachse (32) elektromotorisch oder mittels mindestens eines Magneten oder durch ein Hebelgetriebe oder ein Nockengetriebe bewegt wird.

## Claims

1. Analysis apparatus with an analysis device for analysis of body fluids, and with a magazine for test elements (11, 21) with a fresh supply container (10) and a transfer unit (30) which comprises a transfer element (31) on the circumferential surface (33) of which at least one aperture (34) is formed for receiving a test element (11, 21), wherein a waste container (20) is provided in which used test elements (21) are stored again after use, wherein the transfer element (31) is configured to remove a test element (21) from the fresh supply container (10), deliver it to a work position and convey it into the waste container (20), **characterized in that** the analysis apparatus is configured in such a way that the transfer element (31) is moved automatically from the rest position only when a seal (15) of the fresh supply container (10) has first been lifted off, wherein the seal (15) is located on an end face of a sleeve-shaped receptacle in which the fresh supply container (10) is received, wherein the seal (15) is placed onto the circumferential surface (33) of the transfer element (31).

2. Analysis apparatus according to Claim 1, **characterized in that** the fresh supply container (10), the waste container (20) and the transfer unit (30) are component parts of the analysis device.

3. Analysis apparatus according to Claim 1, **characterized in that** the fresh supply container (10), the waste container (20) and the transfer element (31) are component parts of an exchangeable magazine.

4. Analysis apparatus according to Claim 1, **characterized in that** the waste container (20) has, on its open side, at least one stripper element (23) for removing a used test element (21) from its work position (34).

5. Analysis apparatus according to Claim 5, **characterized in that** the stripper element (23) reaches into a recess (38) formed on the circumferential surface (33) of the transfer element (31).

6. Analysis apparatus according to one of the preceding claims, **characterized in that** the transfer element (31) is pivotable about a pivot axis (32) and is held in a rest position by a spring (45, 48) with moment jump.

7. Analysis apparatus according to the preceding claim, **characterized in that** the rest position of the transfer element (31) of the transfer unit (30) is defined by a position in which an aperture (34) for receiving a test element (11) lies in a work position between the opening of the fresh supply container (10) and the opening of the waste container (20).

8. Analysis apparatus according to one or more of the preceding claims, **characterized in that** the transfer unit (30) is driven by means of actuators (49, 50) that have a shape-memory alloy.

9. Analysis apparatus according to one of the preceding claims, **characterized in that** the analysis apparatus is configured in such a way that the fresh supply container (10) is opened by actuators (49, 50) that also drive the transfer element (31).

10. Analysis apparatus according to one of the preceding claims, **characterized in that** the analysis apparatus is configured in such a way that the seal (15) automatically sits back on the circumferential surface (33) only when the transfer element (31) is located again in a rest position.

11. Analysis apparatus according to one of the preceding claims, **characterized in that** the analysis apparatus is configured in such a way that the transfer element (31) is moved about its pivot axis (32) by electric motor or by means of at least one magnet or by a lever mechanism or a cam mechanism.

## Revendications

1. Dispositif d'analyse avec un appareil d'analyse pour l'analyse de liquides corporels et avec un magasin pour des éléments de test (11, 21) avec un stock frais (10) et une unité de transfert (30), qui comprend un élément de transfert (31), dont la surface périphérique (33) comporte au moins une découpe (34) destinée à recevoir un élément de test (11, 21), dans lequel il est prévu un récipient à déchets (20), dans lequel des éléments de test consommés (21) sont récoltés après leur utilisation, dans lequel l'élément de transfert (31) est conçu pour prélever un élément de test (21) dans le stock frais (10), l'amener à une position de traitement et l'envoyer dans le récipient à déchets (20), **caractérisé en ce que** le dispositif d'analyse est conçu de telle manière que l'élément de transfert (31) ne soit déplacé automatiquement à partir de la position de repos que lorsqu'un joint d'étanchéité (15) du stock frais (10) a été soulevé au préalable, dans lequel le joint d'étanchéité (15) se trouve sur un côté frontal d'un récipient en forme de douille, dans lequel le stock frais (10) est logé, dans lequel le joint d'étanchéité (15) est appliqué sur la surface périphérique (33) de l'élément de transfert (31).

2. Dispositif d'analyse selon la revendication 1, **caractérisé en ce que** le stock frais (10), le récipient à déchets (20) et l'unité de transfert (30) sont des composants de l'appareil d'analyse.

3. Dispositif d'analyse selon la revendication 1, **caractérisé en ce que** le stock frais (10), le récipient à déchets (20) et l'élément de transfert (31) sont des composants d'un magasin remplaçable.

4. Dispositif d'analyse selon la revendication 1, **caractérisé en ce que** le récipient à déchets (20) présente à son extrémité ouverte au moins un élément de raclage (23) pour soulever un élément de test consommé (21) hors de sa position de traitement (34).

5. Dispositif d'analyse selon la revendication 5, **caractérisé en ce que** l'élément de raclage (23) pénètre dans un évidement (38) formé sur la surface périphérique (33) de l'élément de transfert (31).

6. Dispositif d'analyse selon l'une quelconque des revendications précédentes, **caractérisé en ce que** l'élément de transfert (31) peut pivoter autour d'un axe de pivotement (32) et est maintenu en position de repos au moyen d'un ressort (45, 48) avec saut instantané.

7. Dispositif d'analyse selon la revendication précédente, **caractérisé en ce que** la position de repos de l'élément de transfert (31) de l'unité de transfert (30) est définie par une position, dans laquelle une découpe (34) destinée à recevoir un élément de test (11) se trouve dans une position de traitement entre l'ouverture du stock frais (10) et l'ouverture du récipient à déchets (20).

8. Dispositif d'analyse selon une ou plusieurs des revendications précédentes, **caractérisé en ce que** l'unité de transfert (30) est entraînée au moyen d'actionneurs (49, 50), qui présentent un alliage à mémoire de forme.

9. Dispositif d'analyse selon l'une quelconque des revendications précédentes, **caractérisé en ce que** le dispositif d'analyse est conçu de telle manière que le stock frais (10) soit ouvert par des actionneurs (49, 50), qui entraînent également l'élément de transfert (31).

10. Dispositif d'analyse selon l'une quelconque des revendications précédentes, **caractérisé en ce que** le dispositif d'analyse est conçu de telle manière que le joint d'étanchéité (15) ne soit à nouveau appliqué automatiquement sur la surface périphérique (33) que lorsque l'élément de transfert (31) se trouve de nouveau dans une position de repos.

11. Dispositif d'analyse selon l'une quelconque des revendications précédentes, **caractérisé en ce que** le dispositif d'analyse est conçu de telle manière que l'élément de transfert (31) sont déplacé autour de son axe de pivotement (32) par un moteur électrique ou au moyen d'au moins un aimant ou au moyen d'un mécanisme à leviers ou d'un mécanisme à cames.
